# EUROPEAN PATENT APPLICATION

(11) **EP 1 398 617 A1**
(43) Date of publication of application: **17.03.2004**
(21) Application number: 03255711.8
(22) Date of filing: 12.09.2003
(51) Int. Cl.: G01N 21/27, G01M 15/00, G01N 33/00, G01N 21/31

(54) **Exhaust gas sensor**

(30) Priority: 13.09.2002 GB 0221221
(71) Applicant: Delphi Technologies, Inc., Troy, MI 48007 (US)
(72) Inventor: Kubis, Rudiger, 54329 Konz (DE)
(74) Representative: Keltie, David Arthur

(57) **Abstract**

An exhaust gas sensor for measuring at least one constituent gas element within an exhaust flow includes a gas sample cell (12) for receiving a gas sample. An emitter module (22) is provided including an emitter means (40a, 40b; 44; 50) for emitting radiation at one or more wavelength and a first fibre optic coupling (24, 26) for transmitting radiation from the emitter module (22) to the gas sample cell (12). A second fibre optic coupling (34) transmits radiation emitted from the gas sample cell to a detection module (35). The detection module (35) includes a detection means (36) for detecting radiation transmitted through the gas sample cell (12) at the or each wavelength and for providing a detector output signal indicative of the intensity of detected radiation at the one or more wavelength, so as to permit measurement of the concentration of at least one constituent element of the gas sample. It is a beneficial property of the exhaust gas sensor that it comprises means (64) for compensating for temperature variations with the gas sample cell (12). For example the sensor may include means, such as a heat exchanger (64), for maintaining the temperature of the gas sample within the gas sample cell (12) at a substantially constant value.

## Description

The invention relates to an exhaust gas sensor for measuring constituent gas elements of an exhaust stream or flow from a vehicle. In particular, but not exclusively, the invention relates to an exhaust gas sensor suitable for use on board a vehicle.

Commercially available exhaust gas sensors are available and facilitate the measurement of constituent elements of an exhaust stream (for example CO, CO₂, NOx, HC). It is a requirement that such devices are mounted within the exhaust stream and thus it is inevitable that they are prone to thermal strain due to the high temperatures of the exhaust. Such thermal strain, together with mechanical strains that are suffered, have a detrimental effect on the reliability of such devices. Installation of such devices and in-situ adjustment is also inconvenient.

It is an object of the present invention to provide an exhaust gas sensor, which is capable of measuring the amount of at least one constituent gas element or component within an exhaust flow and overcomes or alleviates the aforementioned problems.

According to a first aspect of the present invention, there is provided an exhaust gas sensor for measuring at least one constituent gas element within an exhaust flow, the sensor comprising:
a measurement cell for receiving exhaust,
an emitter module including an emitter means for emitting radiation at one or more wavelength and a first fibre optic coupling for transmitting radiation from the emitter module to the measurement cell,
a detection module including a second fibre optic coupling through which radiation emitted from the measurement cell is transmitted to a detection means, the detection means being arranged to detect radiation at the or each wavelength and provide a detector output signal representative of the intensity of radiation at the or each wavelength for comparison with predetermined absorption characteristic data,
the gas sensor further comprising means for compensating for temperature variations within the measurement, thereby to ensure a substantially independent measurement of the concentration of constituent gas elements is obtained.

The sensor preferably includes or is used in conjunction with a computer processor means for storing predetermined absorption characteristic data of one or more gas elements, and including means for calculating the concentrations of the gas elements within the sample in response to the or each detector output signal.

In one preferred embodiment, the processor means is arranged to receive a signal to provide an indication of the temperature of the gas within the cell and includes means for compensating for temperature variations within the gas within the cell in response to said temperature.

In one preferred embodiment, the gas sensor includes a temperature sensor for measuring the temperature of the exhaust within the measurement cell, and the processor means is provided with means for adjusting the measured concentrations of the gas elements in dependence upon the measured temperature, thereby to determine a substantially temperature independent measurement of said concentration(s).

The gas sensor may also include a pressure sensor for measuring the pressure of the gas sample within the measurement cell, and wherein the computer processor is provided with means for adjusting the measured concentrations of the gas elements in dependence upon the measured pressure, thereby to determine a substantially pressure independent measurement of said concentration(s).

In an alternative preferred embodiment, the exhaust gas sensor may include means for maintaining the temperature of the gas sample within the gas sample cell at a substantially constant value.

For example, the gas sensor may include a heat exchanger for maintaining the temperature of the gas sample within the gas sample cell at a substantially constant value.

It is an advantage of the invention that any variations in temperature, which would otherwise prejudice the accuracy of the measured concentrations, can be substantially removed or avoided altogether. The compensation process is either carried out prior to determination of the concentrations, by maintaining the temperature of the exhaust within the measurement cell at a substantially constant value, or after the concentrations have been determined by adjusting the measured concentrations in dependence upon temperature.

A further advantage of the invention is that, as the emitter means and the detection means can both be decoupled from the measurement cell, problems associated with mechanical and thermal strain are reduced and the accuracy of measurement is improved. The sensor is therefore particularly suitable for use on board a vehicle, as it is substantially unaffected by vibrations.

By virtue of the fibre optic couplings, the emitter and detection means can be mounted in an accessible location on the vehicle to make in situ adjustment or replacement relatively easy. Moreover, the emitter and detection means may be located remotely from the measurement cell and from the engine exhaust pipe, to reduce problems which may otherwise be encountered due to the high temperatures of the exhaust.

In one embodiment, the emitter means includes a source of radiation for emitting radiation within a range of wavelengths.

The detection means may preferably include a plurality of detector elements, wherein each detector element is arranged to provide an output signal indicative of the intensity of detected radiation at a different wavelength within the wavelength range.

For example, each detector may have an associated filter for selectively transmitting radiation at a respective wavelength within the wavelength range.

As each one of the detectors is sensitive to radiation at a different wavelength, the detector output signals can be used to determine the concentration of different constituents of the gas sample if the absorption characteristics of the different constituents are known.

For example, the emitter means and the detection means may be configured to permit measurement of the concentration of CO, CO₂, NOx, HC and smoke within the exhaust flow.

In an alternative embodiment, the emitter means may include a plurality of radiation sources, each for emitting radiation at a different wavelength.

In this embodiment the detection means may include a single detector arranged to detect radiation at each wavelength, and whereby the source of radiation is arranged to provide pulsed radiation at a plurality of different wavelengths.

Alternatively, a plurality of detector elements may be provided, each detector element being arranged to detect radiation at a respective wavelength within the wavelength range.

In this embodiment, each detector may have an associated filter for selectively transmitting radiation at the respective wavelength within the wavelength range.

In embodiments for which a plurality of detector elements are used, the elements may be comprised in a common array, with each of the filters, if provided, being associated with a respective element or portion of the array.

In one embodiment, the emitter means may include one or more sources, for example laser diodes, for emitting radiation at one or more wavelengths. For example, the emitter module may include a plurality of pigtail laser diodes. Such an arrangement of pigtail laser diodes is found to be particularly robust, and thus makes the gas sensor particularly suitable for use on board a vehicle.

The first and/or second fibre optic coupling may, for example, take the form of chalcogenide infrared (CIR) fibre or polycrystalline infrared (PIR) fibre. In a preferred embodiment, the first and/or the second fibre optic coupling includes a region of thermally insulating optical fibre, for example sapphire fibre, which is preferably arranged adjacent to the measurement cell.

The provision of a length of sapphire fibre (or other thermally insulating fibre) near or adjacent to the measurement cell provides the advantage any thermal damage, which may otherwise occur to the fibre optic couplings and the emitter/detection means due to the very high temperatures of exhaust gas within the cell, is minimised.

In one embodiment, the measurement cell may be located, at least in part, within a main exhaust pipe of an associated engine. Preferably, the emitter module and the detection module may be incorporated within a common unit or module.

A surface of the measurement cell may have infra red reflection properties such that radiation passing unabsorbed from the emitter module through the cell and to the surface is reflected back through the cell to the detection module.

The surface of the measurement cell may be provided with a reflective coating or may be formed from a reflective material.

The sensor may further include an inlet flow means for delivering a gas sample from the exhaust flow to the measurement cell, typically in the form of an inlet pipe, which may be provided with a heat exchanger to maintain the temperature of the exhaust within the cell at a substantially constant value.

For the purpose of this specification, the absorption characteristic of a gas element shall be taken to mean the absorption coefficient of the element as a function of wavelength. It will be appreciated that such absorption characteristic data may also be stored in the processor as a function of gas temperature and/or pressure.

The exhaust gas sensor may be provided with one or more of a pressure transducer or a temperature sensor for measuring the pressure and/or temperature of the gas sample within the cell, and may be used in combination with a computer processor or engine controller provided with means for adjusting the detector output signals in dependence upon the measured temperature and/or pressure of the gas sample, thereby to determine a substantially constant temperature and/or pressure.

According to a second aspect of the invention, there is provided a method for measuring at least one constituent gas element within an exhaust flow, comprising transmitting radiation at one or more wavelength through a first fibre optic coupling to a measurement cell, transmitting radiation which passes unabsorbed through the measurement cell through a second fibre optic coupling to a detector arrangement, detecting radiation transmitted through the measurement cell at the or each wavelength, providing a detector output signal indicative of the intensity of detected radiation at the one or more wavelength, comparing the detector output signal with predetermined absorption characteristic data, compensating for temperature variations within the measurement cell (12), and determining a substantially independent measurement of the concentration of the or each constituent gas element from said comparison.

In one embodiment of the method, the step of compensating for temperature variations within the measurement cell is carried out prior to the determination step.

For example, the method may include measuring the temperature of the exhaust within the measurement cell and controlling application of a heating effect to the exhaust in the measurement cell in response to the measured temperature, and maintaining the temperature of the exhaust within the cell at a substantially constant amount.

Alternatively, the step of compensating for temperature variations within the measurement cell may be carried out subsequent to the determination step.

For example the step of compensating for temperature variations within the measurement cell may include measuring the temperature of exhaust within the measurement cell (12), and adjusting the calculated concentration of the or each constituent gas element in dependence upon the measure temperature.

Preferred and/or optional features of the sensor of the first aspect of the invention may be provided for the method of the second aspect of the invention, in accordance with the accompanying claims 1 to 18.

The invention will now be described, by way of example only, with reference to the accompanying drawings in which:
Figure 1 is a schematic diagram of a first type of exhaust gas sensor,
Figure 2 is an enlarged view of a part of the gas sensor in Figure 1,
Figure 3 is an enlarged view of a part of an alternative gas sensor to that shown in Figure 1,
Figure 4 shows example measurements of (a) the intensity of radiation transmitted to a gas sample of the exhaust gas sensor in Figure 1, and (b) the intensity of radiation transmitted through the gas sample and from which the constituent elements of the gas sample may be deduced,
Figure 5 illustrates example measurements for a two-gas sensitive exhaust sensor, and
Figures 6 to 9 are further alternative embodiments of the exhaust gas sensor to that shown in Figures 1 to 3.

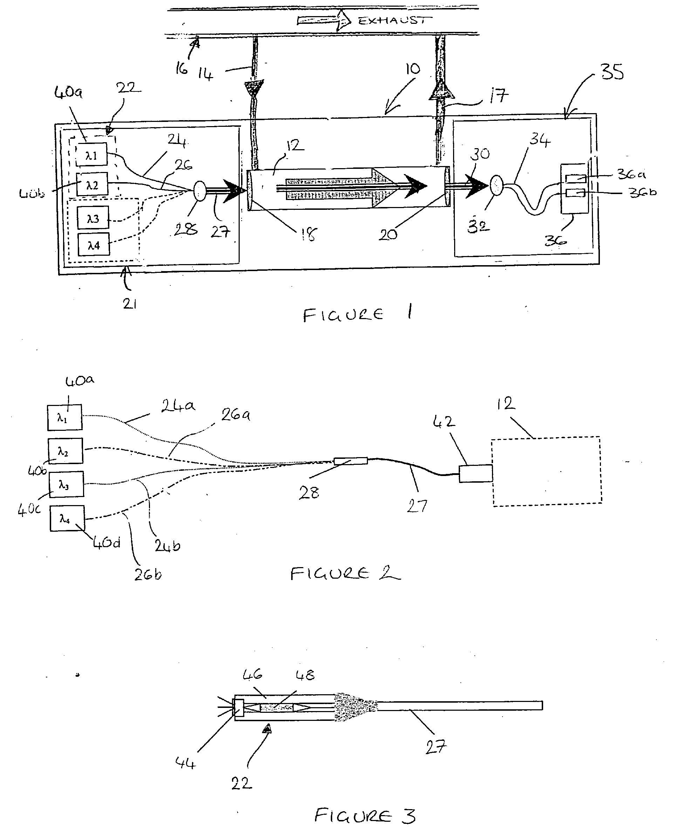

Figure 1 shows an exhaust gas sensor 10 including a gas measurement cell or cavity 12, which is arranged to receive a gas sample from an exhaust stream through a inlet pipe 14. The inlet pipe 14 communicates with the main exhaust pipe 16 of the associated engine so that, in use, a sample of gas flows from the main exhaust pipe 16, through the inlet pipe 14, through the cell 12 and to a gas outlet pipe 17 for return to the bulk exhaust flow. The cell 12 includes inlet and outlet windows, 18, 20 respectively, at respective inlet and outlet ends of the cell 12.

An emitter module 21 is provided at the inlet end of the cell 12 and a detection module 35 is provided at the outlet end of the cell 12. The emitter module includes a radiation source 22 for emitting radiation at a plurality of wavelengths (e.g. λ1 and λ2) and for transmitting radiation through respective fibre optic lengths 24 (λ1) and 26 (λ2) of the module 21 to the cell 12. The fibre optic lengths 24, 26 are coupled through a lens or collimator 28 to a first end of a further inlet fibre optic length 27, the other end of which is coupled to the inlet window 18 of the cell 12.

Radiation transmitted through the cell 12 exits through the outlet window 20, and is coupled to a first outlet fibre optic length 30 of the detection module 35. One end of the first outlet fibre optic length 30 is coupled to the outlet window 20 of the cell 12 and the other end of the first outlet fibre optic length 30 is coupled to a further lens or collimator 32. Collimated radiation is transmitted through additional outlet fibre optic lengths 34 to a detection arrangement 36 having two detector elements 36a, 36b. The detector arrangement 36 is arranged to detect radiation which passes unabsorbed through the cell 12.

In one implementation, the source 22 emits radiation at two wavelengths, λ1 and λ2. A proportion of emitted radiation is transmitted along the length of the cell 12, through the cell outlet window 20, through the first outlet fibre optic length 30, the collimator 32 and the additional fibre optic lengths 34 to the two detector elements 36a, 36b. Each of the detector elements is sensitive to radiation at a different one of the wavelengths, λ1 and λ2. Gases and/or other particulate material within the cell absorb the radiation at different wavelengths depending on the constituents thereof. Therefore, by monitoring the wavelengths of the radiation detected by the detector elements 36a, 36b, the wavelengths absorbed, and hence the constituents of the gas or particulate material, can be determined. If the absorption profile of a particular constituent is known, the measurement of the intensity of transmitted radiation at a given wavelength λ1, λ2 (i.e. the intensity of radiation which is transmitted through the cell 12 at each of the wavelengths, λ1 or λ2) can be used to determine the relative strength of that particular constituent within the sample.

Radiation emitted from the source 22 may include, for example, wavelengths λ1, λ2 falling within the range of 3 to 5.5 µm. The detector elements 36a, 36b provide output signals indicative of the intensity of radiation at wavelengths λ1 and λ2 respectively. The detector output signals are provided to a processor or computer (not shown) for data manipulation to determine the relative strengths of different gas elements within the cell 12 based on the known absorption characteristics of said elements, which are pre-stored within the processor.

As indicated by the dashed lines in Figure 1, in an alternative arrangement the source 22 may be extended to include third and fourth (or higher) radiation emitters at wavelengths λ3 and λ4 respectively, with corresponding means for detecting the additional wavelengths being provided in the detection module 35.

It is a general requirement that, in order to measure the intensity of each wavelength transmitted through the cell 12, the detection module 35 must be capable of detecting each of those wavelengths (e.g. λ1 and λ2 in the first example). This may be achieved by providing the detection module 35 with a detector arrangement 36a, 36b having two separate detector elements 36a, 36b, as shown in Figure 1 and as described previously. Alternatively the detector arrangement 36 may include just a single detector element which is sensitive to both wavelengths λ1 and λ2 and by providing a source 22 of pulsed radiation at the two emission wavelengths λ1 and λ2. By appropriate programming of the processor, a measurement of transmitted radiation at each wavelength, λ1 and λ2, can then be deduced.

As the exhaust gas sample within the cell 12 reaches relatively high temperatures, it is advantageous if at least a portion of both the inlet and outlet fibre optic lengths 27, 30, which are adjacent to the cell 12, is formed from a thermally insulting fibre. For example, sapphire fibre may be used as it has good insulating properties. Sapphire fibre also has a relatively high attenuation coefficient for wavelengths over 4 µm, and so if the source 22 emits radiation in the wavelength region of 3 to 5.5 µm it is desirable if the length of any sapphire fibre region of fibres 27 and 30 is relatively short

Regions of the inlet and outlet fibres 27, 30 and/or the other fibres 24, 26, 34 may take the form of chalcogenide infrared (CIR) fibre, suitable for transmitting radiation in the range of between 2 and 6 µm and having an operating temperature range of, for example, 280-400 K. Alternatively, polycrystalline infrared (PIR) fibre may be used, suitable for transmitting radiation in the range of between 4 and 18 µm and having an operating temperature range of, for example, 210-470 K.

As the emitter means 22 and the detection means 36 are both decoupled from the measurement cell 12, problems associated with mechanical and thermal strain are reduced and the accuracy of measurement is improved considerably. Furthermore, by using fibre optic couplings between the emitter/detection means 22, 36 and the cell 12, the emitter and detection means 22, 36 can be mounted in conveniently accessible positions.

Figure 2 illustrates a part of a practical arrangement of the sensor shown in Figure 1, in which the source 22 includes four sources 40a-40d of radiation at wavelengths λ1 to λ4. The corresponding fibre optic lengths 24a, 24b, 26a, 26b are coupled to an optical coupler 28, the output of which is coupled to a length 27 of sapphire fibre. A connector 42 (e.g. SMA) is used as the interface to the measurement cell 12 to couple the inlet fibre 27 to the cell 12. The fibre optic lengths 24a, 24b for transmitting wavelengths λ1 and λ3 may be of a first type (e.g. CIR fibre) and the fibre optic lengths 26a, 26b for transmitting wavelengths λ2 and λ4 may be of a second type (e.g. PIR), if desired, depending on the wavelengths λ1-λ4 and the transmission/thermal properties of the fibre optic materials.

Referring to Figure 3, in an alternative arrangement the source 22 may include a plurality of laser diodes 44 (only one of which is shown) mounted within a module housing 46 and arranged to emit radiation into a collimator lens 48 mounted internally within the housing 46. The output from the lens 48 is coupled to a corresponding inlet fibre optic length 27. The arrangement takes the form of a so-called "pigtail" miniature laser diode, and provides a particularly robust means of transmitting radiation from the emitter module to the cell 12 with relatively low attenuation (i.e. high performance).

In a further alternative arrangement (not shown), the detection module 35 may include a detector arrangement 36 having four separate detectors, each being provided with a filter for selectively transmitting only one of the wavelengths λ1-λ4, and for preventing transmission of the other wavelengths. The sensor may be extended to measure radiation intensities at more or less wavelengths, λ1-λn.

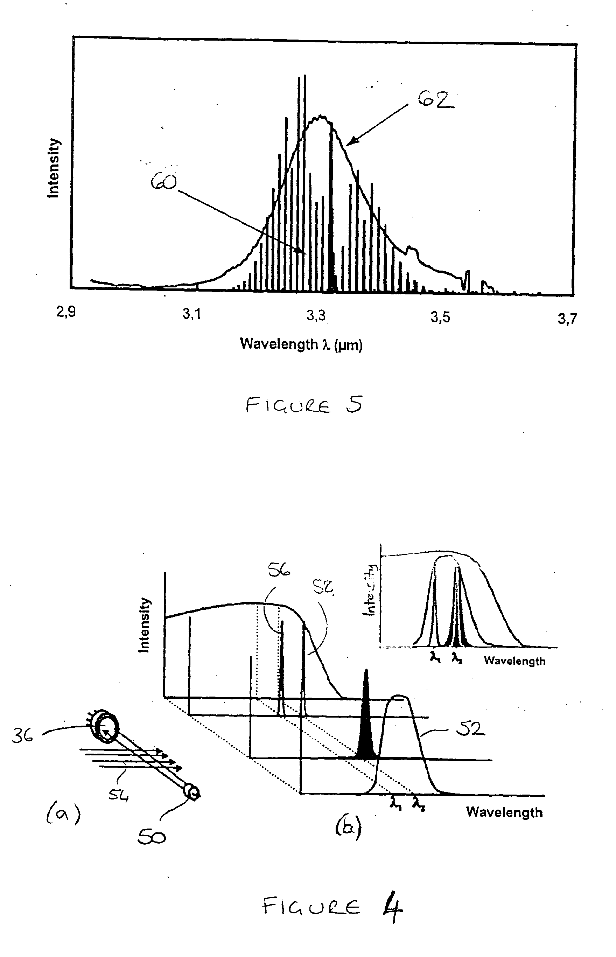

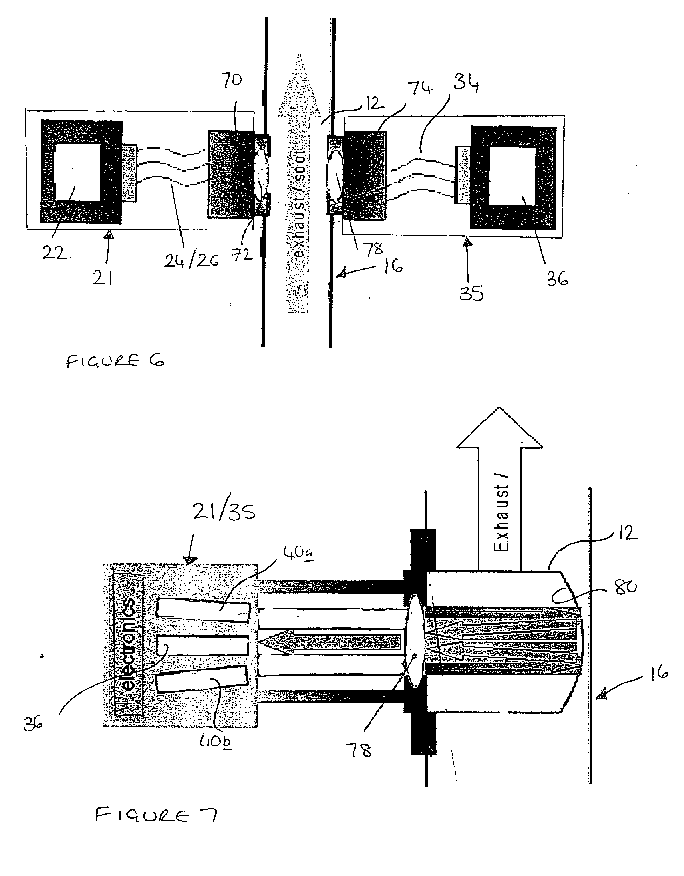

Figure 4(a) illustrates a further alternative arrangement, in which the emitter means includes a broadband source 50 of radiation emitting radiation within a wavelength band 52 (seen in Figure 4(b)). The detector arrangement 36 includes two detector elements (not identified), each comprising a detector and an associated filter for transmitting one of the wavelengths, λ1 or λ2, but for substantially preventing transmission of the other of the wavelengths. The gas flow through the cell 12 is indicated by arrows 54, and produces an intensity profile 56, 58 at the detector arrangement 36 at wavelengths λ1 and λ2 respectively. Figure 5 is a plot to show an example of the absorption characteristic 60 of the gas sample within the cell 12 (for example, methane), and the broadband emission spectrum 62 for the source 50.

In Figure 6, the measurement cell 12 is incorporated within the main exhaust pipe 16. In this arrangement the requirement for the inlet sample pipe 14 is avoided. Radiation emitted from the emitter module 21 is transmitted through the fibre optic lengths 24/26 and is coupled through an inlet optical interface (e.g. collimator) 70 and an inlet lens 72 provided in the wall of the pipe 16 and, thus, into the cell 12 of the pipe 16. Radiation which passes unabsorbed through the gas flow within the pipe 16 passes through an outlet lens 78 provided in an oppositely facing region of the pipe wall, and is coupled through an output optical interface 74 (e.g. collimator) into additional fibre optic lengths 34 and, hence, to the detection arrangement 36.

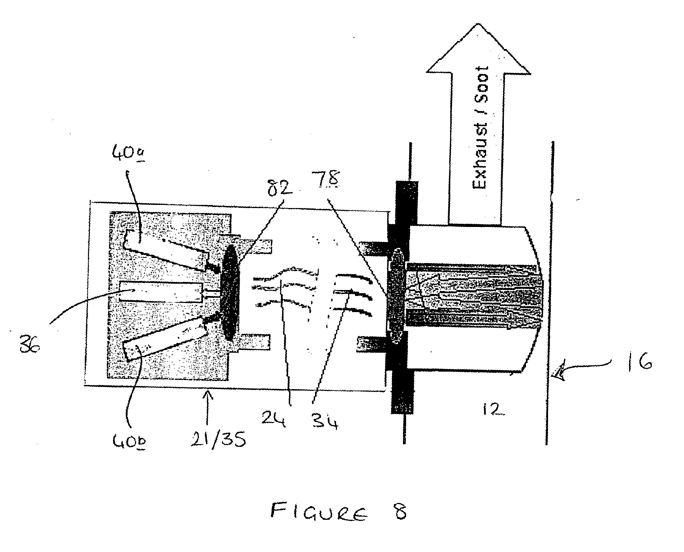

A further alternative arrangement of the sensor is shown in Figure 7, in which again the measuring cell 12 is incorporated within the main exhaust pipe 16. In this case, however, the emitter and detection functions (provided by 40a, 40b and 36 respectively) are incorporated within a common emitter/detection unit or module 21/35. An internal surface 80 of the measuring cell 12 is provided with an infra red reflective coating, or may be formed from an infra red reflective material, such that radiation passing unabsorbed through the cell 12 and being incident upon the surface 80 is reflected back through the cell 12 to the module 21/35. Radiation emitted from the emitter/detection module 21/35 is transmitted through free space to a collimating lens 78 provided in the wall of the exhaust pipe 16. Radiation reflected by the surface 80 passes back through the cell 12, through the lens 78 and through free space to the emitter/detection module 21/35.

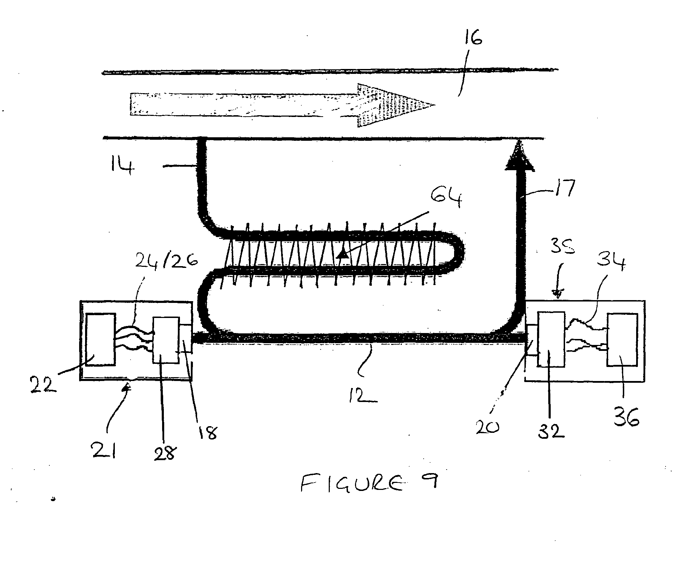

The embodiment of the exhaust gas sensor in Figure 8 is similar to that shown in Figure 7, except that input and output optical fibres 24, 34 respectively are provided to transmit radiation to and from the emitter/detection module 21/35 through the measurement cell 12. In this arrangement an additional lens 82 is provided, which serves to couple radiation from the sources 40a, 40b to the inlet fibres 24 and from the outlet fibres 34 to the detector arrangement 36.

It is a feature of the present invention that compensation is made for any variation in the absorption characteristics of the gas sample with temperature. At higher gas temperatures, for example, the measured intensities of radiation transmitted through the cell 12 may indicate a lower concentration of a particular gas element than is actually present. In order to obtain a more accurate, near temperature-independent measurement of the concentration of constituent gas elements, the temperature of the gas sample may be measured and a corresponding adjustment made to either the measured intensity of detected radiation or the calculated concentration(s) to compensate for the temperature dependency. This may be achieved in software by reference to pre-calibrated data relating the absorption characteristic to temperature, which data may be stored in the form of a look-up table or a data map.

Similarly, the absorption characteristics of the gas elements will have some gas pressure dependence, and so a similar adjustment may be made to the measured intensity of detected radiation or the calculated concentration(s) to deduce a near-pressure dependent measurement. Again, this may be implemented in software by reference to pre-calibrated data relating the absorption characteristic of the constituent gas elements to pressure, which data may be stored in a look-up table or a data map.

The temperature and/or pressure compensation techniques are applicable to any of the embodiments of the gas sensor described previously with reference to Figures 1 to 8.

As an alternative compensation means, the gas sample may be "pre-conditioned" to substantially eliminate the effects of temperature variation in the measured gas concentrations. Figure 9 shows a similar sensor arrangement to that shown in Figure 1, although the inlet and outlet fibres 27, 30 adjacent to the cell 12 are not shown in this example.

Similar parts in Figure 9 to those described with reference Figure 1 are denoted with like reference numerals. So, for example, the emitter module 21 on the inlet side of the cell 12 includes a source 22 coupled through fibre optic lengths 24, 26 to a collimator 28 and subsequently to the inlet window 18 of the cell 12. The detection module 35 on the outlet side of the cell 12 includes outlet fibre optic lengths 34 for coupling radiation transmitted through the collimator 32 from the cell 12 to the detector arrangement 36.

In the arrangement of Figure 9, the gas sample pipe 14 is provided with a heat exchanger 64 to provide a degree of thermal stability. The heat exchanger 64 may take the form of an electrical heater which is heated by means of the associated vehicle power supply. A current is supplied to the heat exchanger 64 to raise the temperature of the gas sample pipe 14. A temperature sensor (not shown) for measuring the temperature of the gas in the cell 12 is also provided. The level of current applied to the heat exchanger 64, and the time for which the current is applied, are determined in software in response to the measured temperature so as to heat or cool the exhaust sample within the cell 12 such that it is maintained at a substantially constant temperature. Before any calculation is performed using the detector output signals, the gas sample is "pre-conditioned" by activating the heat exchanger 64 to re-establish the desired, constant temperature.

In practice, it may be advantageous to applying a heating effect to gas in the sample pipe 14 (e.g. by supplying a current to the heat exchanger 64) immediately following engine start-up and/or at low or extremely low ambient temperatures, so as to reduce condensation of water on the pipe 14. Deposition of water on the optical components of the gas sensor 10, for example on the inlet and outlet windows 18, 20, reduces the intensity of radiation at the detection module 35, and should preferably be substantially eliminated, for example by periodically heating the inlet pipe 14 or by heating the pipe 14 under particularly cold conditions. Alternatively, by pre-calibrating the sensor to allow for water deposits within the system, the detector output signals can be adjusted in software to compensate for condensation effects.

When required, the heat exchanger 64 may be cooled by means of cooling fluid circulating through the engine cooling system. It has been found that when the engine is working at its normal operating temperature, a coolant temperature of approximately 110°C provides a substantially consistent temperature of the gas within the cell 12. Typically, at normal engine operating temperatures, the temperature of gas within the cell 12 is between 90°C and 200°C, depending on the gas sample pipe length and engine load.

It is preferable to locate the exhaust gas sensor in a position having as low an ambient temperature as possible. This is particularly beneficial if the radiation sources 40a-40d and detectors of the detection module 35 are semiconductor devices which operate most satisfactorily at low temperatures.

It will be appreciated that in the aforementioned examples the emitter and detection means, 22, 36 form part of respective emitter and detection modules 21, 35, which also include respective fibre optic couplings, 24, 26, 27 and 30, 34. This may provide a convenient means of manufacture, as the emitter and detection aspects of the sensor are formed in modular units. However, the emitter means 22 and detection means 36 need not be formed as part of modular units with their respective fibres optic couplings, and in some circumstances it may be preferred if the fibre optic couplings are manufactured separately, to provide a greater flexibility in locating the emitter and detection parts 22, 36.

## Claims

1. An exhaust gas sensor for measuring at least one constituent gas element within an exhaust flow, the sensor comprising:
a measurement cell (12) for receiving the exhaust flow,
an emitter module (22) including an emitter means (40a, 40b; 44; 50) for emitting radiation at one or more wavelength and a first fibre optic coupling (24, 26, 27) for transmitting radiation from the emitter module (22) to the measurement cell (12),
a detection module (35) including a second fibre optic coupling (34, 30) through which radiation emitted from the measurement cell (12) is transmitted to a detection means (36), the detection means (36) being arranged to detect radiation at the or each wavelength and provide a detector output signal representative of the intensity of radiation at the or each wavelength for comparison with predetermined absorption characteristic data,
the gas sensor further comprising means (64) for compensating for thermal variations within the measurement cell (12), thereby to ensure a substantially independent measurement of the concentration of the or each constituent gas element is obtained.

2. The exhaust gas sensor as claimed in claim 1, comprising processor means for storing predetermined absorption characteristic data of one or more gas elements, and including means for calculating the concentrations of the gas elements within the sample in response to the or each detector output signal.

3. The exhaust gas sensor as claimed in claim 2, including a temperature sensor for measuring the temperature of the exhaust within the measurement cell (12), and wherein the processor means is provided with means for adjusting the or each measured concentration in dependence upon the measured temperature, thereby to determine a substantially temperature independent measurement of said concentration(s).

4. The exhaust gas sensor as claimed in claim 2 or claim 3, including a pressure sensor for measuring the pressure of the exhaust within the measurement cell (12), and wherein the processor means is provided with means for adjusting the or each measured concentration in dependence upon the measured pressure, thereby to determine a substantially pressure independent measurement of said concentration(s).

5. The exhaust gas sensor as claimed in claim 1 or claim 2, including means (64) for maintaining the temperature of the exhaust within the measurement cell (12) at a substantially constant value.

6. The exhaust gas sensor as claimed in claim 5, comprising a heat exchanger (64) for maintaining the temperature of the exhaust within the measurement cell (12) at a substantially constant value.

7. The exhaust gas sensor as claimed in claim 6, wherein the heat exchanger (64) is arranged within an inlet flow means (14) for providing the exhaust to the measurement cell (12).

8. The exhaust gas sensor as claimed in any one of claims 1 to 7, wherein the emitter means (22) includes a source of radiation for emitting radiation within a range of wavelengths.

9. The exhaust gas sensor as claimed in claim 8, wherein the detection means (35) includes a plurality of detector elements (36a, 36b), wherein each detector element is arranged to provide an output signal indicative of the intensity of detected radiation at a respective wavelength within the wavelength range.

10. The exhaust gas sensor as claimed in claim 9, wherein each detector element (36a, 36b) has an associated filter for selectively transmitting radiation at the respective wavelength within the wavelength range.

11. The exhaust gas sensor as claimed in any one of claims 1 to 7, wherein the emitter means (22) include a plurality of radiation sources (40a, 40b, 40c, 40d), each for emitting radiation at a different wavelength or within a different sub-band of wavelengths.

12. The exhaust gas sensor as claimed in claim 11, wherein the detection means (36) includes a single detector arranged to detect radiation at each wavelength, or within each sub-band of wavelengths, and whereby the source of radiation is arranged to provide pulsed radiation at a plurality of different wavelengths.

13. The exhaust gas sensor as claimed in claim 11, wherein the detection means (36) includes a plurality of detector elements (36a, 36b), each detector element being arranged to detect radiation at a respective wavelength within the wavelength range.

14. The exhaust gas sensor as claimed in claim 13, wherein each detector element (36a, 36b) has an associated filter for selectively transmitting radiation at the respective wavelength within the wavelength range.

15. The exhaust gas sensor as claimed in any one of claims 1 to 14, wherein the emitter means includes one or more laser diodes (44) for emitting radiation at one or more wavelength.

16. The exhaust gas sensor as claimed in any one of claims 1 to 15, wherein the first and/or the second fibre optic coupling (24, 27, 34, 30) includes a region (27, 30) of thermally insulating optical fibre.

17. The exhaust gas sensor as claimed in any one of claims 1 to 16, wherein the emitter module and the detection module are incorporated within a common unit or module (21/35).

18. The exhaust gas sensor as claimed in any one of claims 1 to 17, wherein the measurement cell (12) is located, at least in part, within a main exhaust pipe (16) of an associated engine.

19. A method for measuring at least one constituent gas element within an exhaust flow, the method comprising;
transmitting radiation at one or more wavelength through a first fibre optic coupling (24, 26, 27) to a measurement cell (12),
transmitting radiation which passes unabsorbed through the measurement cell (12) through a second fibre optic coupling (30, 34) to a detector arrangement (36),
detecting radiation transmitted through the measurement cell (12) at the or each wavelength,
providing a detector output signal indicative of the intensity of detected radiation at the one or more wavelength,
comparing the detector output signal with predetermined absorption characteristic data,
compensating for temperature variations within the measurement cell (12), and
determining a substantially independent measurement of the concentration of the or each constituent gas element from said comparison.

20. The method as claimed in claim 19, where in the step of compensating for temperature variations within the measurement cell (12) is carried out prior to the determination step.

21. The method as claimed in claim 20, including:
measuring the temperature of the exhaust within the measurement cell (12) and controlling application of a heating effect to the exhaust in the measurement cell (12) in response to the measured temperature, and thus
maintaining the temperature of the exhaust within the cell (12) at a substantially constant amount.

22. The method as claimed in claim 19, wherein the step of compensating for temperature variations within the measurement cell (12) is carried out subsequent to the determination step.

23. The method as claimed in claim 22, wherein the step of compensating for temperature variations within the measurement cell (12) includes:
measuring the temperature of exhaust within the measurement cell (12), and
adjusting the calculated concentration of the or each constituent gas element in dependence upon the measure temperature.
